# EUROPÄISCHE PATENTSCHRIFT

(11) **EP 0 878 183 B1**
(45) Veröffentlichungstag und Bekanntmachung des Hinweises auf die Patenterteilung: **23.03.2005**
(21) Anmeldenummer: 98108579.8
(22) Anmeldetag: 12.05.1998
(51) Int. Cl.: A61K 6/083, C09J 133/06, A61K 6/00

(54) **Adhäsivsystem für dentale Zwecke**
Adhesive system for dental use
Système adhésif pour soins dentaires

(30) Priorität: 12.05.1997 DE 19719890
(43) Veröffentlichungstag der Anmeldung: 18.11.1998
(73) Patentinhaber: 3M ESPE AG, 82229 Seefeld (DE)
(72) Erfinder: Stefan, Klaus-Peter, Dr., 82229 Seefeld (DE)

(56) Entgegenhaltungen:
- EP-A- 0 219 058
- WO-A-93/12760
- DE-A- 2 312 559
- DE-A- 4 105 550
- US-A- 4 182 035
- US-A- 5 133 957
- US-A- 5 498 643

## Beschreibung

Die vorliegende Erfindung betrifft ein Adhäsivsystem zur dauerhaften Befestigung von dentalen Restaurationsmaterialien, wie Composite-Füllmaterialien, Inlays, Onlays, Kronen, Brücken, Keramiken, Veneers und Maryland-Brücken an der Zahnhartsubstanz.

Bekannte Systeme, um derartige Restaurationsmaterialien mit Zähnen zu verbinden, bestehen in der Regel aus drei Komponenten. Komponente 1 enthält eine Säure (z.B. Phosphorsäure oder Maleinsäure) um den Zahnschmelz und das Dentin anzuätzen, Komponente 2 enthält hydrophile vernetzungsfähige Verbindungen, die gut an die Zahnhartsubstanz anfließen, und Komponente 3 enthält vernetzungsfähige Substanzen, die sich gut mit Komponente 2 verbinden, aber deutlich hydrophober sind als Zahnschmelz und Dentin. Meist enthält Komponente 3 noch einen oder mehrere Photoinitiatoren, wodurch das Adhäsivsystem durch Bestrahlen mit Licht gehärtet wird. Derartige Vorbehandlungen zum adhäsiven Verbund von Zahnhartsubstanz mit Restaurationsmaterialien sind beispielsweise in U. Blunck, Quintessenz (1996), 47 (1), 19-35; R. Frankenberger, N. Krämer, J. Sindel, Dtsch. Zahnärztl. Z. (1996), 51, 556-560; und A. Pagliarini, R. Rubini, M. Rea, C. Campese, R. Grandini, Quintessence International (1996), 27, 265-270; beschrieben. Wie diesen Literaturzitaten zu entnehmen ist, verbinden sich auf diese Weise vorbehandelte Zahnoberflächen gut mit den entsprechenden Dentalmaterialien und führen zu einem dauerhaften Verbund zwischen Restaurationsmaterial und Zahn.

In den vergangenen Jahren wurden auch Adhäsivsysteme entwickelt, welche die Komponenten 2 und 3 in einer Komponente zusammenfassen, um den Gesamtaufwand für die Befestigungsprozedur zu reduzieren, wie z.B. in den EP-A-0 234 934 und 0 305 083 beschrieben.

Nachteilig bei den beschriebenen lichthärtenden Adhäsivsystemen ist, daß die dabei auftretende Dicke der Befestigungsschicht nicht genau kontrolliert werden kann und bedingt durch die Aushärtung mittels Bestrahlung nicht mehr flexibel ist. Dies führt z.B. beim Einsetzen eines Inlays oder Onlays zu einer unerwünschten Erhöhung der Präparation und beeinträchtigt damit die Paßgenauigkeit erheblich. Wie der ISO-Norm 9917 zu entnehmen ist, führen bereits Schichtdicken des Adhäsivsystems von mehr als 25 µm zu nicht mehr akzeptablen Paßungenauigkeiten, die für den Zahnarzt einen erheblichen Mehraufwand an Einschleifarbeiten bedeuten bzw. in ungünstigen Fällen (z.B. durch Brechen eines Inlays bei der Bißkontrolle) eine Neuanfertigung der zahntechnischen Arbeit erforderlich machen.

Um diesen Mangel zu beheben, wurden Adhäsivsysteme entwickelt, die anstelle der zuvor beschriebenen Komponenten 2 oder 3 zwei Teilkomponenten 2a und 2b bzw. 3a und 3b beinhalten, die vor ihrem Aufbringen auf die Zahnhartsubstanz miteinander gemischt werden (siehe z.B. "Clearfil Line Bond 2" in Reality now, 1996, 80, 2). Durch dieses Vermischen wird ein chemischer Aushärtungsprozeß gestartet, der das gesamte Adhäsivsystem innerhalb einiger Minuten zur Aushärtung bringt. Bis zum endgültigen Aushärten verbleibt dem Behandler in der Regel aber noch genügend Zeit, um das Restaurationsmaterial auf die Präparation aufzubringen, in der das Adhäsivsystem noch genügend flexibel ist, um eine ausreichende Paßgenauigkeit zu erreichen. Eventuelle Überschüsse des Adhäsivsystems können am Präparationsrand herausgedrückt werden.

Nachteilig bei diesen Adhäsivsystemen ist ihr relativ aufwendiger Applikationsprozeß, denn im Vergleich zu den eingangs beschriebenen strahlungshärtenden Systemen werden hier noch eine Komponente und ein Mischschritt mehr benötigt (Komponente 2 bzw. 3 besteht aus zwei räumlich voneinander getrennt aufzubewahrenden Massen 2a und 2b bzw. 3a und 3b). Dies kann leicht zu Verwechslungen beim Zahnarzt führen und damit die Haftkraft des Systems nachhaltig schädigen. Ein weiterer Nachteil dieses Adhäsivsystems ist die Abhängigkeit des Behandlers von der Abbindezeit des jeweiligen Befestigungssystems, die zu unnötigen Wartezeiten während der Behandlung führen oder bei schneller abbindenden Systemen ein vorzeitiges Aushärten des Adhäsivs mit sich bringen kann, mit den gleichen Nachteilen wie bei den lichthärtenden Systemen.

Aufgabe der vorliegenden Erfindung ist es daher, ein einfach anzuwendendes, während des Eingliederns eines Restaurationsmateriales flexibles und anschließend schnell aushärtendes Adhäsivsystem zur Verfügung zu stellen, welches hohe Haftkräfte und einen dauerhaften Verbund zwischen dentalen Restaurationsmaterialien und der Zahnhartsubstanz gewährleistet.

Gelöst wird diese Aufgabe durch die Bereitstellung eines Adhäsivsystems, das aus folgenden Bestandteilen besteht:
i) einer Komponente, in Form einer dünnen Schicht die mindestens einen Aktivator, der einen Bestandteil eines chemischen Initiatorsystems darstellt, sowie mindestens eine ethylenisch ungesättigte Verbindung, welche zur Polymerisation befähigt ist, und gegebenenfalls ein Lösungsmittel für den Aktivator und die ungesättigte Verbindung enthält, darüber
ii) einer Komponente, in Form einer dünnen Schicht die mindestens eine ethylenisch ungesättigte Verbindung enthält, welche zur Polymerisation befähigt ist, sonst aber keinen weiteren Aktivator enthält, der mit dem Aktivator aus Komponente i) eine chemische Aushärtung starten kann, und darüber
iii) einem dentalen Restaurationsmaterial, welches mindestens einen Aktivator enthält, der zusammen mit dem Aktivator aus i) eine Polymerisationsreaktion starten kann, bzw. einem Zement zur Befestigung von schon ausgehärteten dentalen Restaurationsmaterialien, welcher mindestens einen Aktivator enthält, der zusammen mit dem Aktivator aus Komponente i) eine radikalische Polymerisation starten kann.
   In einer bevorzugten Ausführungsform umfaßt das erfindungsgemäße Adhäsivsystem zusätzlich
iv) eine saure Komponente, die Zahnschmelz und Dentin anätzt und zur Vorbehandlung dient.

Bei der Anwendung dieses Adhäsivsystems geht der Behandler so vor, daß er zuerst Komponente iv) auf die Zahnhartsubstanz aufträgt, eine bestimmte Zeit einwirken läßt und dann mit Wasser wieder abspült, so wie dies auch bei anderen Bondingsystemen als Vorbehandlung üblich ist. Als nächstes wird auf die Zahnhartsubstanz Komponente i) in Form einer dünnen Schicht aufgetragen. Darüber wird eine dünne Schicht von Komponente ii) aufgetragen, und als letzter Schritt wird das dentale Restaurationsmaterial iii) auf die Schichten aus i) und ii) gedrückt bzw. der Zement iii) aufgebracht und auf diesen das schon ausgehärtete dentale Restaurationsmaterial gedrückt. Durch dieses Aufdrücken der Komponente iii) erfolgt eine Vermischung der Komponenten i), ii) und iii), wodurch die beiden Aktivatorkomponenten des Initiatorsystems miteinander in Kontakt kommen und den Aushärtungsprozeß starten. Überraschenderweise genügt bereits einfaches Aufdrücken der Komponente iii), um eine derartige Durchmischung zu erzielen, daß der Aushärteprozeß in vollem Umfang gestartet wird und vergleichbar gute Haftwerte wie mit herkömmlichen Adhäsivsystemen erreicht werden, wie in den Beispielen belegt ist.

Der Vorteil für den Anwender besteht darin, daß er die einzelnen Bestandteile des erfinderischen Adhäsivsystems i), ii) und iii) nur einzeln nacheinander aufzutragen braucht und schließlich den Beginn der Härtungsreaktion durch Aufbringen des Restaurationsmaterials selbst steuern kann. Ein zwischenzeitliches Belichten mit den oben beschriebenen Nachteilen oder ein zeitaufwendiges und umständliches Zusammenmischen zweier Komponenten entfällt bei dieser Vorgehensweise.

Im folgenden wird die Erfindung näher beschrieben und durch Beispiele weiter erläutert.

Die Komponente i) ist vorzugsweise ein Gemisch aus einem geeigneten Lösungsmittel, welches kurzkettige polare ethlyenisch ungesättigte Verbindungen, die zur Polymerisation befähig sind, zu lösen vermag und einem Aktivator, der Bestandteil eines chemischen Initiatorsystems ist. Als chemische Initiatorsysteme werden hierbei solche verstanden, die durch Vermischen von mindestens zwei chemisch unterschiedlichen Substanzen ohne weitere Energiezufuhr Radikale bilden können, welche dann eine Polymerisationsreaktion auszulösen vermögen. Beispiele für derartige chemische Initiatorsysteme sind Peroxy-Amin- oder Peroxy-Protonenspender-Metallverbindungs-Gemische, wie sie z.B. von J. M. Antonucci et al. in J. Dental Research, (1979), 58 (9), S. 1887-1889 oder in der US-A-5 166 117 sowie in den EP-A-0 115 410, 0 115 948, 0 120 559 und 0 277 413 beschrieben sind. Komponente i) enthält dann beispielsweise entweder ein Amin oder eine Peroxy-Verbindung oder einen entsprechenden Protonenspender. Weiterhin ist in Komponente i) mindestens eine ethylenisch ungesättigte Verbindung enthalten, die in der Lage ist, radikalisch polymerisiert werden zu können.

Komponente i) muß dann kein Lösungsmittel enthalten, wenn die eingesetzten Bestandteile der Komponente i) per se genügend fließfähig und hydrophil sind. Bevorzugt aber ist der Einsatz eines Lösungsmittels. Mögliche Lösungsmittel für die Komponente i) sind kurzkettige Alkohole, kurzkettige Ketone, aliphatische oder ungesättigte Ether, cyclische Ether sowie Carbonsäuren und Dicarbonsäuren. Besonders bevorzugte Lösungsmittel sind Wasser, Ethanol und Aceton.

Denkbare Amine sind Alkylarylamine, Dialkylarylamine, Trialkylamine oder Derivate davon. Besonders bevorzugte Amine sind N,N-Bis-β-oxyethyl-3,5-di-t-butylanilin, N,N-Bis-β-oxyethyl-4-toluidin, N,N-Bis-ß-hydroxyethyl-3,4,5-trimethylanilin sowie die in der DE-A-2 658 538 beschriebenen Amine.

Als Peroxy-Verbindungen können beispielsweise Diacylperoxide, Perester, Perketale, Peroxydicarbonate, Dialkylperoxide, Ketonperoxide oder Alkylhydroxyperoxide verwendet werden. Besonders bevorzugte Peroxy-Verbindungen sind Di-t-butylperoxid, t-Butylperoxybenzoat, Di-p-methylbenzoylperoxid, Dibenzoylperoxid sowie Dilauroylperoxid.

Geeignete Protonenspender sind Säuren, Aminosäuren, Phenole oder Hydroxyalkene. Besonders bevorzugte Protonenspender sind Sulfinsäuren, Barbitursäure, Thiobarbitursäure und Ascorbinsäure.

Verwendbare Metalle, als Zusatz um die Reaktivität des Protonenspenders zu steigern, sind Kupfer, Silber, Cer, Eisen, Kobalt, Nickel, Vanadium und Mangan. Ebenso geeignet sind entsprechende Metallsalze oder Organo-Metallverbindungen, wie sie z.B. in der EP-A-0 732 098 beschrieben sind.

Als ethylenisch ungesättigte Verbindung kommen solche in Frage, die mindestens eine radikalisch polymerisierbare Doppelbindung enthalten. Dies können mono- oder polyfunktionelle Acrylate und Methacrylate sein, wie sie beispielsweise in der EP-A-0 480 472 beschrieben sind. Ebenfalls verwendbar sind weiter funktionalisierte Monomere mit endständigen Acrylatoder Methacrylatgruppen wie sie z.B. in der DE-A-2 312 559 und in der EP-A-0 219 058 beschrieben sind. Selbstverständlich können auch Gemische dieser Monomeren verwendet werden.

Der Gehalt an Aktivator, gegebenenfalls gelöst im Lösungsmittel von Komponente i), beträgt zwischen 1 und 40 Gew.-%, bevorzugt zwischen 5 und 20 Gew.-%, bezogen auf das Gesamtgewicht der Komponente i).

Der Gehalt an polymerisierbaren Verbindungen, gegebenenfalls gelöst im Lösungsmittel von Komponente i), beträgt zwischen 5 und 80 Gew.-%, bevorzugt zwischen 10 und 60 Gew.-%, bezogen auf das Gesamtgewicht der Komponente i).

Daneben kann Komponente i) noch übliche Stabilisatoren und Hilfsstoffe enthalten.

Die Komponente ii) des erfindungsgemäßen Adhäsivsystems enthält mindestens eine ethylenisch ungesättigte Verbindung, die in der Lage ist, radikalisch polymerisiert werden zu können. Dies können mono- oder polyfunktionelle Acrylate und Methacrylate sein, wie sie beispielsweise in der EP-A-0 480 472 beschrieben sind. Ebenfalls verwendbar sind weiter funktionalisierte Monomere mit endständigen Acrylat- oder Methacrylatgruppen, wie sie z.B. in der DE-A-2 312 559 und in der EP-A-0 219 058 beschrieben sind. Selbstverständlich können auch Gemische dieser Monomeren verwendet werden. Daneben kann Komponente ii) noch übliche Stabilisatoren und Hilfsstoffe enthalten. Wichtig ist, daß Komponente ii) keinerlei Aktivatoren enthält, die zusammen mit dem Aktivator aus Komponente i) Radikale bilden können. Übliche Photoinitiatoren, wie sie in dentalen Massen zur Anwendung kommen, können in Komponente i) jedoch enthalten sein.

Die Komponente iii) ist ein dentales Restaurationsmaterial, das mindestens einen Aktivator enthält, der zusammen mit dem Aktivator aus Komponente i) eine radikalische Polymerisation starten kann, oder ein Zement zur Befestigung von schon ausgehärteten dentalen Restaurationsmaterialien, der mindestens einen Aktivator enthält, der zusammen mit dem Aktivator aus Komponente i) eine radikalische Polymerisation starten kann.

Mögliche Aktivatoren der Komponente iii) sind solche wie sie für Komponente i) beschrieben worden sind bzw. Gemische dieser Aktivatoren. Für den Fall, daß in Komponente iii) ein komplettes Initiatorsystem vorhanden ist, muß die Komponente iii) aus zwei räumlich voneinander getrennten Teilen bestehen, die unmittelbar vor dem Aufbringen von Komponente iii) auf Komponente ii) miteinander vermischt werden. Der eine Teil von Komponente iii) enthält dann beispielsweise eine Peroxy-Verbindung, der andere Teil ein Amin.

Dentale Restaurationsmaterialien, welche bereits Aktivatoren enthalten können, sind übliche Composite-Füllmaterialien. Diese können entweder lichthärtend sein und liegen meist in Form einer einzigen Paste vor, oder sie besitzen einen Selbsthärtungsmechanismus und bestehen dann aus zwei räumlich voneinander getrennt vorliegenden Pasten.

Andere dentale Restaurationsmaterialien, die lange vor ihrer adhäsiven Befestigung an der Zahnhartsubstanz endgehärtet werden, wie z.B. Inlays, Kronen, Brücken, Veneers oder Maryland-Brücken, müssen über einen Zement mit dem Adhäsivsystem verbunden werden. Diese Zemente können auch Composite-Massen sein, wie sie z.B. in einer Übersicht von McComb, "Adhesive luting cements - classes, criteria and usage", Compendium (1996), 17, 759-773; beschrieben sind, müssen aber genügend fließfähig sein bzw. durch geeignete Methoden, wie z.B. Ultraschall (beschrieben in EP-A-0 480 472), genügend fließfähig gemacht werden, um Zwischenräume zwischen Restaurationsmaterial und Zahnhartsubstanz optimal ausfüllen zu können.

Sowohl beim Aufbringen des Restaurationsmaterials, welches bereits mindestens einen Aktivator enthält, der mit dem Aktivator aus Komponente i) reagieren kann, als auch beim Aufbringen von dentalen Restaurationen über einen Zement, wird auf die Schichten des erfindungsgemäßen Adhäsivsystems ein Druck ausgeübt. Dieser Druck führt zumindest zu einer partiellen Durchmischung der Komponenten i), ii) und iii), wodurch die Aktivatoren der Komponenten i) und iii) miteinander in Kontakt kommen und eine radikalische Polymerisation des gesamten Adhäsivsystems in Gang setzen. Dabei wird auch die Komponente ii), die keine Aktivatoren enthält, polymerisiert.

Die vorzugsweise vorhandene Komponente iv) des erfindungsgemäßen Systems enthält einen sauren Bestandteil, wie er üblicherweise bei dentalen Vorbehandlungen zum Einsatz kommt. So sind beispielsweise von M. Buonocore in "The Challenge of Bonding to Dentin", The Acid Etch Technique, (St. Paul 1974), Säuren beschrieben, die sich zu diesem Zweck eignen. Komponente iv) kann als Flüssigkeit oder in Form eines Gels zum Einsatz kommen. Kommerziell erhältlich Produkte die als Komponente iv) verwendet werden können, sind beispielsweise Ätzgel Minitip® (Fa. Espe, Seefeld), oder Esitcid®-G-Gel (Fa. Kulzer, Hanau). Die Komponente iv) ist in der Regel und vorzugsweise wäßrige Phosphorsäure.

Die oben beschriebene Vorgehensweise führt zu einem guten Haftverbund zwischen Zahnhartsubstanz und Restaurationsmaterial, wie in den folgenden Beispielen, welche die Erfindung noch genauer beschreiben sollen, belegt wird.

### Beispiele

### Referenzbeispiel 1:

### Herstellungsbeispiel für Komponente i) ohne Aktivator:

40 g entionisiertes Wasser, 50 g Hydroxyethylmethacrylat (HEMA) und 10 g Mono-(2-methacroyloxy-ethyl)-Mg-Phosphat (Mg-HEMA-Phosphat) werden solange miteinander gemischt, bis eine klare Lösung i-0) entsteht.

### Beispiel 1

### Herstellungsbeispiel für Komponente i):

39 g entionisiertes Wasser, 48,75 g Hydroxyethylmethacrylat (HEMA), 9,75 g Mono-(2-methacroyloxy-ethyl)-Mg-Phosphat (Mg-HEMA-Phosphat) und 2,5 g N,N-Bis-β-hydroxyethyl-3,4,5-trimethylanilin (ODBA) werden so lange miteinander gemischt, bis eine klare Lösung i-1) entsteht.

### Beispiel 2

### Herstellungsbeispeil für Komponente i):

38 g entionisiertes Wasser, 47,5 g Hydroxyethylmethacrylat (HEMA), 9,5 g Mono-(2-methacroyloxy-ethyl)-Mg-Phosphat (Mg-HEMA-Phosphat) und 5 g N,N-bis-β-hydroxyethyl-3,4,5-trimethylanilin (ODBA) werden so lange miteinander gemischt, bis eine klare Lösung i-2) entsteht.

### Beispiel 3

### Herstellungsbeispiel für Komponente i):

37 g entionisiertes Wasser, 46,25 g Hydroxyethylmethacrylat (HEMA), 9,25 g Mono-(2-methacroyloxy-ethyl)-Mg-Phosphat (Mg-HEMA-Phosphat) und 7,5 g N,N-Bis-β-hydroxyethyl-3,4,5-trimethylanilin (ODBA) werden so lange miteinander gemischt, bis eine klare Lösung i-3) entsteht.

### Beispiel 4

### Herstellungsbeispiel für Komponente i):

36 g entionisiertes Wasser, 45 g Hydroxyethylmethacrylat (HEMA), 9 g Mono-(2-methacroyloxy-ethyl)-Mg-Phosphat (Mg-HEMA-Phosphat) und 10 g N,N-Bis-β-hydoxyethyl-3,4,5-trimethylanilin (ODBA) werden so lange miteinander gemischt, bis eine eine klare Lösung i-4) entsteht.

### Beispiel 5

### Herstellungsbeispiel für Komponente ii):

40 g 2,2-Bis-4-(3-hydroxypropoxy-phenyl)-propandimethacrylat, 25 g Triethylenglykoldimethacrylat (TEGDMA), 18 g 2-(10'-Methacryloxy-decyl)-malonsäure, 10 g Isopropyliden-bis-[2-hydroxy-3-(4-phenoxy)-propyl]-methacrylat und 7 g Hydroxyethylmethacrylat (HEMA) werden so lange miteinander gemischt, bis eine homogene Mischung entsteht.

### Beispiel 6

### Herstellung eines Befestigungszementes:

63,5 g Glaspulver (Fa. Schott), welches zuvor gemäß dem in der EP-A-0 649 887 beschriebenen Verfahren silanisiert wurde, 17,2 g Quarzpulver, welches zuvor gemäß dem in der EP-A-0 649 887 beschriebenen Verfahren silanisiert wurde, 3,7 g OX50 (Kieselsäure Fa. Degussa), welches ebenfalls nach dem vorstehenden Verfahren silanisiert wurde, 14,8 g Yttriumtrifluorid und 0,8 g Di-p-methylbenzoylperoxid werden zu einer homogenen Pulvermischung vermengt.

29,2 g 2,2-Bis-4-(3-hydroxypropoxy-phenyl)-propandimethacrylat, 69,2 g Bishydroxymethyl-tricyclo[5.2.1.0²,⁶]-decandimethacrylat und 0,6 g N,N-Bis-β-hydroxyethyl-3,4,5-trimethylanilin (ODBA) werden so lange miteinander verrührt, bis eine klare Lösung entsteht.

Unmittelbar vor der Anwendung wird die Pulvermischung mit der flüssigen Komponente im Verhältnis von 2,5:1 innig gemischt.

### Beispiel 7

### Anwendung der Komponenten i)-iv) zur Befestigung eines Inlays mit einem Zement:

Die Bestimmung der Haftfestigkeiten der Restaurationsmaterialien erfolgte durch Haftabzugsversuche von Zahnoberflächen, die durch Präparation von Rinderzähnen freigelegt wurden. Die Vorbehandlung der Zähne geschah dabei wie folgt:

Je 5 nach dem Extrahieren tiefgefrorene Rinderzähne werden aufgetaut, von restlichem Zahnfleisch gereinigt und die Wurzeln durch Absägen mit einer Diamantsäge abgetrennt. Die noch verbliebene Pulpa wird mit Hilfe einer Pulpanadel entfernt und die Zähne dann mit Leitungswasser gespült. Plane Schmelz- bzw. Dentinoberflächen werden durch labiales Schleifen der Zähne an einer wassergekühlten Diamantschleifmaschine erhalten. Die Zähne werden dann so in Silikon eingebettet, daß die abgeschliffene, gut feucht gehaltene Oberfläche nach oben zeigt und auf jeden Zahn wird ein Wachsplättchen aufgeklebt, welches eine Ausstanzung von 6 mm Durchmesser hat.

### Zur Anwendung des erfinderischen Adhäsivsystems wird folgendermaßen vorgegangen:

Auf die durch die Wachsplättchen begrenzten Zahnoberflächen wird eine Schicht Ätzgel Minitip® (Komponente iv); Espe, Seefeld) so aufgebracht, daß eine geschlossene Schicht entsteht. Nach 20 Sekunden Einwirkzeit wird das säurehaltige Gel gründlich mit Wasser abgespült.

Anschließend wird mit Hilfe eines kleinen Pinsels die in Beispiel 1, 2, 3 oder 4 bzw. in Referenzbeispiel 1 beschriebene Komponente i) auf die Zahnoberfläche aufgebracht, so daß die gesamte durch das Wachsplättchen begrenzte Oberfläche damit benetzt wird, und läßt diese Schicht 20 Sekunden auf die Oberfläche einwirken.

Nach kurzem Trockenblasen mit ölfreier Druckluft wird die in Beispiel 5 beschriebene Komponente ii) ebenfalls mit einem Pinsel so auf die Zahnoberfläche aufgetragen, daß die gesamte, durch das Wachsplättchen begrenzte Oberfläche damit bedeckt wird.

Nach 20 Sekunden Einwirkzeit der Komponente ii) wird der zuvor angemischte Zement aus Beispiel 6 als Komponente iii) in einer dünnen Schicht über die Schicht von Komponente ii) aufgebracht. Das zuvor gefertigte Inlay wird in diesem Versuch durch eine Schraube simuliert, die mit dem Kopf auf den Zement aufgedrückt wird. Nach 3 Minuten ist die Aushärtung erfolgt, und die Zähne werden bis zur Haftabzugskraftbestimmung 24 Stunden feucht bei 36°C gelagert. Zur Bestimmung der Haftabzugskraft werden die an den Zahnoberflächen adhäsiv befestigten Schrauben in eine Halterung eingeschraubt und diese wiederum in eine Zwickprüfmaschine vom Typ Zwick 1435 eingespannt und die Kraftmessung durchgeführt.

### Beispiel 8

### Anwendung der Komponenten i)-iv) zur Befestigung einer Composite-Füllmasse:

Die Präparation der Zähne einschließlich des Einsatzes der Komponente iv), sowie die Auftragung der Komponenten i) und ii) erfolgen wie in Beispiel 7 beschrieben. Darauf wird das zuvor angemischte Composite-Füllmaterial Sono-Cem® (Espe, Seefeld) als Komponente iii) aufgebracht und mit einem Kugelstopfer fest auf die Zahnoberfläche gedrückt. Nach etwa 3,5 Minuten ist das Composite und das Adhäsivsystem ausgehärtet. Bis zur Haftmessung werden die befüllten Zähne mindestens 24 Stunden bei 36°C feucht gelagert. Als Haftwert wurden 2,2 ± 0,9 MPa ermittelt.

### Referenzbeispiel 2

### Befestigung einer Compositefüllmasse mit einem lichthärtenden Adhäsivsystem:

Die Vorbehandlung der Zahnoberflächen erfolgt gemäß den Herstellerangaben mit EBS® (Espe, Seefeld). Nach der Lichthärtung wird die Composite-Füllmasse Pertac®-Hybrid (Espe, Seefeld) auf das Adhäsiv aufgebracht und entsprechend den Herstellerangaben lichtgehärtet. Bis zur Haftmessung werden die befüllten Zähne mindestens 24 Stunden bei 36°C feucht gelagert. Als Haftwert werden 6,2 ± 2,3 MPa ermittelt.

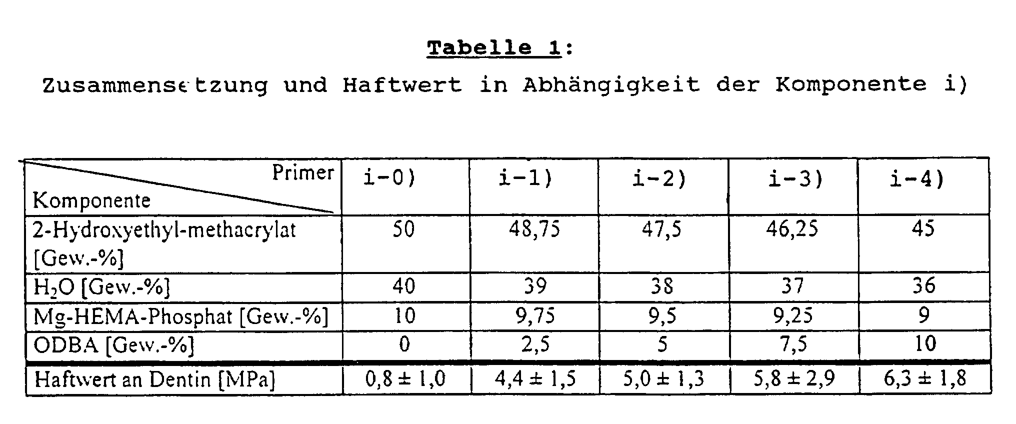

## Patentansprüche

1. Adhäsivsystem zur Befestigung von dentalen Restaurationsmaterialien an der Zahnhartsubstanz bestehend aus
i) einer Komponente in Form einer dünnen Schicht, die mindestens einen Aktivator beinhaltet, der einen Bestandteil eines chemischen Initiatorsystems darstellt, sowie mindestens eine ethylenisch ungesättigte Verbindung enthält, welche zur Polymerisation befähigt ist, darüber
ii) einer Komponente in Form einer dünnen Schicht, die mindestens eine ethylenisch ungesättigte Verbindung enthält, welche zur Polymerisation befähigt ist, sonst aber keinen weiteren Aktivator enthält, der mit dem Aktivator aus Komponente i) eine chemische Aushärtung starten kann, und darüber
iii) einem dentalen Restaurationsmaterial, welches mindestens einen Aktivator enthält, der zusammen mit dem Aktivator aus i) eine Polymerisationsreaktion starten kann, bzw. einem Zement zur Befestigung von schon ausgehärteten dentalen Restaurationsmaterialien, welcher mindestens einen Aktivator enthält, der zusammen mit dem Aktivator aus Komponente i) eine radikalische Polymerisation starten kann.

2. Adhäsivsystem nach Anspruch 1, **dadurch gekennzeichnet, daß** es zusätzlich
iv) eine saure Vorbehandlungskomponente für das Anätzen von Zahnschmelz und Dentin
umfaßt.

3. Adhäsivsystem nach den Ansprüchen 1 oder 2, **dadurch gekennzeichnet, daß** Komponente i) als Aktivator eine Peroxyverbindung oder ein Amin oder einen Protonenspender enthält.

4. Adhäsivsystem nach den Ansprüchen 1 bis 3, **dadurch gekennzeichnet, daß** Komponente i) mindestens eine ethylenisch ungesättigte Verbindung enthält, die mindestens eine radikalisch polymerisierbare Doppelbindung aufweist.

5. Adhäsivsystem nach den Ansprüchen 1 bis 4, **dadurch gekennzeichnet, daß** die Inhaltsstoffe von Komponente i) in einem geeigneten Lösungsmittel gelöst sind.

6. Adhäsivsystem nach den Ansprüchen 1 bis 5, **dadurch gekennzeichnet, daß** der Gehalt an Aktivator in Komponente i) zwischen 1 und 40 Gew.-% beträgt, bezogen auf das Gesamtgewicht der Komponente i).

7. Adhäsivsystem nach den Ansprüchen 1 bis 6, **dadurch gekennzeichnet, daß** der Gehalt an polymerisierbaren Verbindungen in Komponente i) zwischen 5 und 80 Gew.-% beträgt, bezogen auf das Gesamtgewicht der Komponente i).

8. Adhäsivsystem nach den Ansprüchen 1 bis 7, **dadurch gekennzeichnet, daß** Komponente iii) ein Composite-Füllmaterial ist, das licht- oder selbsthärtend ist.

9. Adhäsivsystem nach den Ansprüchen 1 bis 7, **dadurch gekennzeichnet, daß** Komponente iii) ein Zement ist, der selbsthärtend ist.

10. Adhäsivsystem nach den Ansprüchen 1 bis 9 für die Befestigung von Composite-Füllmaterialien, Inlays, Onlays, Kronen, Brücken, Keramiken, Veneers oder Maryland-Brücken als dentale Restaurationsmaterialien an der Zahnhartsubstanz.

11. Verwendung von
i) einer Komponente in Form einer dünnen Schicht, die mindestens einen Aktivator beinhaltet, der einen Bestandteil eines chemischen Initiatorsystems darstellt, sowie mindestens eine ethylenisch ungesättigte Verbindung enthält, welche zur Polymerisation befähigt ist, darüber
ii) einer Komponente in Form einer dünnen Schicht, die mindestens eine ethylenisch ungesättigte Verbindung enthält, welche zur Polymerisation befähigt ist, sonst aber keinen weiteren Aktivator enthält, der mit dem Aktivator aus Komponente i) eine chemische Aushärtung starten kann, und darüber
iii) einem dentalen Restaurationsmaterial welches mindestens einen Aktivator enthält, der zusammen mit dem Aktivator aus i) eine Polymerisationsreaktion starten kann, bzw. einem Zement zur Befestigung von schon ausgehärteten dentalen Restaurationsmaterialien, welcher mindestens einen Aktivator enthält, der zusammen mit dem Aktivator aus Komponente i) eine radikalische Polymerisation starten kann,
zur Herstellung eines Adhäsivsystems zur Befestigung von dentalen Restaurationsmaterialien an der Zahnhartsubstanz.

## Claims

1. Adhesive system for fixing dental restorative materials to the hard substance of the tooth, composed of
i) a component in the form of a thin layer which comprises at least one activator, representing part of a chemical initiator system, and comprises at least one ethylenically unsaturated compound which is capable of polymerization, and
ii) a component in the form of a thin layer which comprises at least one ethylenically unsaturated compound which is capable of polymerization, but otherwise contains no further activator which is able with the activator from component i) to initiate a chemical cure, and also
iii) a dental restorative material which comprises at least one activator which together with the activator from i) is able to initiate a polymerization reaction, and/or a cement for fixing dental restorative materials which have already cured, which comprises at least one activator which together with the activator from component i) is able to initiate a free-radical polymerization.

2. Adhesive system according to Claim 1, **characterized in that** it further comprises
iv) an acidic pretreatment component for the etching of enamel and dentine.

3. Adhesive system according to Claim 1 or 2, **characterized in that** component i) comprises as activator a peroxy compound or an amine or a proton donor.

4. Adhesive system according to Claims 1 to 3, **characterized in that** component i) comprises at least one ethylenically unsaturated compound which has at least one free-radically polymerizable double bond.

5. Adhesive system according to Claims 1 to 4, **characterized in that** the constituents of component i) are in solution in an appropriate solvent.

6. Adhesive system according to Claims 1 to 5, **characterized in that** the amount of activator in component i) is between 1% and 40% by weight, based on the total weight of component i).

7. Adhesive system according to Claims 1 to 6, **characterized in that** the amount of polymerizable compounds in component i) is between 5% and 80% by weight, based on the total weight of component i).

8. Adhesive system according to Claims 1 to 7, **characterized in that** component iii) is a composite filling material which is photocuring or self-curing.

9. Adhesive system according to Claims 1 to 7, **characterized in that** component iii) is a cement which is self-curing.

10. Adhesive system according to Claims 1 to 9 for fixing composite filling materials, inlays, onlays, crowns, bridges, ceramics, veneers or Maryland bridges as dental restorative materials to the hard substance of the tooth.

11. Use of
i) a component in the form of a thin layer which comprises at least one activator, representing part of a chemical initiator system, and comprises at least one ethylenically unsaturated compound which is capable of polymerization, and
ii) a component in the form of a thin layer which comprises at least one ethylenically unsaturated compound which is capable of polymerization, but otherwise contains no further activator which is able with the activator from component i) to initiate a chemical cure, and also
iii) a dental restorative material which comprises at least one activator which together with the activator from i) is able to initiate a polymerization reaction, and/or a cement for fixing dental restorative materials which have already cured, which comprises at least one activator which together with the activator from component i) is able to initiate a free-radical polymerization
for preparing an adhesive system for fixing dental restorative materials to the hard substance of the tooth.

## Revendications

1. Système adhésif pour la fixation de matériaux de restauration dentaire sur la substance dure de la dent, constitué par
i) un composant sous forme d'une couche mince, qui contient au moins un activateur qui représente un constituant d'un système d'initiateur chimique, ainsi qu'au moins un composé éthyléniquement insaturé, qui est apte à une polymérisation, en outre
ii) un composant sous forme d'une couche mince qui contient au moins un composé éthyléniquement insaturé, qui est apte à la polymérisation, mais qui ne contient toutefois pas d'autre activateur qui peut démarrer un durcissement chimique avec l'activateur du composant i) et en outre
iii) un matériau de restauration dentaire, qui contient au moins un activateur qui peut démarrer une réaction de polymérisation avec l'activateur i), ou, selon le cas, un ciment pour la fixation de matériaux de restauration dentaire déjà durcis, qui contient au moins un activateur qui peut démarrer une polymérisation radicalaire avec l'activateur du composant i).

2. Système adhésif selon la revendication 1, **caractérisé en ce qu'**il comprend en outre
iv) un composant acide de prétraitement pour attaquer l'émail et la dentine.

3. Système adhésif selon la revendication 1 ou 2, **caractérisé en ce que** le composant i) contient comme activateur un composé peroxy ou une amine ou un donneur de protons.

4. Système adhésif selon les revendications 1 à 3, **caractérisé en ce que** le composant i) contient au moins un composé éthyléniquement insaturé qui présente au moins une double liaison polymérisable par voie radicalaire.

5. Système adhésif selon les revendications 1 à 4, **caractérisé en ce que** les constituants du composant i) sont dissous dans un solvant approprié.

6. Système adhésif selon les revendications 1 à 5, **caractérisé en ce que** la teneur en activateur dans le composant i) est de 1 à 40% en poids, par rapport au poids total du composant i).

7. Système adhésif selon les revendications 1 à 6, **caractérisé en ce que** la teneur en composés polymérisables dans le composant i) est de 5 à 80% en poids par rapport au poids total du composant i).

8. Système adhésif selon les revendications 1 à 7, **caractérisé en ce que** le composant iii) est un matériau de remplissage composite qui est durcissable à la lumière ou auto-durcissable.

9. Système adhésif selon les revendications 1 à 7, **caractérisé en ce que** le composant iii) est un ciment auto-durcissable.

10. Système adhésif selon les revendications 1 à 9 pour la fixation de matériaux de remplissage composites, d'inlays, d'onlays, de couronnes, de bridges, de céramiques, de facettes ou de bridges Maryland comme matériaux de restauration dentaire sur la substance dure des dents.

11. Utilisation
i) d'un composant sous forme d'une couche mince, qui contient au moins un activateur qui représente un constituant d'un système d'initiateur chimique, ainsi qu'au moins un composé éthyléniquement insaturé, qui est apte à une polymérisation, en outre
ii) d'un composant sous forme d'une couche mince qui contient au moins un composé éthyléniquement insaturé, qui est apte à la polymérisation, mais qui ne contient toutefois pas d'autre activateur qui peut démarrer un durcissement chimique avec l'activateur du composant i) et en outre
iii) d'un matériau de restauration dentaire, qui contient au moins un activateur qui peut démarrer une réaction de polymérisation avec l'activateur i), ou, selon le cas, un ciment pour la fixation de matériaux de restauration dentaire déjà durcis, qui contient au moins un activateur qui peut démarrer une polymérisation radicalaire avec l'activateur du composant i)
pour la préparation d'un système adhésif destiné à la fixation de matériaux de restauration dentaire sur la substance dure des dents.
